# EUROPEAN PATENT SPECIFICATION

(11) **EP 1 628 575 B1**
(45) Date of publication and mention of the grant of the patent: **17.11.2010**
(21) Application number: 04731442.2
(22) Date of filing: 06.05.2004
(51) Int. Cl.: A61B 6/12

(54) **APPARATUS FOR NAVIGATING A CATHETER**
GERÄT ZUR NAVIGATION EINES KATHETERS
APPAREIL PERMETTANT DE SONDER AU MOYEN D'UN CATHETER

(30) Priority: 21.05.2003 EP 03101457
(43) Date of publication of application: 01.03.2006
(73) Proprietor: Philips Intellectual Property & Standards GmbH, 20099 Hamburg (DE); Koninklijke Philips Electronics N.V., 5621 BA Eindhoven (NL)
(72) Inventor: TIMINGER, H. Philips Int. Prop. & Standards Gmbh, 52066 Aachen (DE); BORGERT, Jörn Philips Int. Prop.& Standards Gmbh, 52066 Aachen (DE); KRÜGER, Sascha Philips Int. Prop.& Standards Gmbh, 52066 Aachen (DE)
(86) International application number: PCT/IB2004/050603
(87) International publication number: WO 2004/103182

(56) References cited:
- WO-A-99/43253
- WO-A1-00/16684
- US-A- 5 592 939
- US-A1- 2002 049 375
- US-A1- 2002 172 328
- US-A1- 2003 074 011
- US-B1- 6 473 635

## Description

The invention relates to a navigation system for navigating a catheter in a vascular system which is subject to a cyclic intrinsic movement.

During a catheter examination carried out for diagnostic or therapeutic purposes, it is extremely important for the treating physician to know the current position of the instrument (catheter tip, guidewire, etc.) in the patient's vascular system in as precise a manner as possible. In clinical practice, this aim is usually achieved in that the movement of the catheter in the patient's body is subject to X-ray monitoring, wherein possibly radio-opaque markers are applied to the catheter. In order to have on the X-ray images also a best possible representation of the course of the vessel, an X-ray contrast agent is moreover injected from time to time. However, this procedure has the disadvantage of being a relatively high strain on the patient on account of the X-ray radiation and the contrast agent and - in terms of the X-ray radiation - also on the medical staff.

For the reasons given above, a catheter navigation is desired in which only a few X-ray images of the vascular system have to be taken with administration of a contrast agent and the catheter is then monitored on these static images or "road maps". The current spatial position and orientation of the catheter must in this case be determined by suitable methods such as, for example, location by means of a magnetic field. However, such a procedure using static road maps runs into problems when the monitored body volume moves and therefore no longer geometrically coincides with the static road maps. An overall movement of the patient may in this connection be practically ruled out as a cause of error by measures such as careful instruction, steady positioning and sedation of the patient. However, cyclic intrinsic movements of the organs of the body by breathing and heartbeat cannot be avoided. These movements are obviously particularly disruptive during examinations of organs in the chest area, such as the coronary vessels.

In this respect, DE 199 46 948 A1 discloses a method which tries to achieve better position accuracy by using a number of road maps which are taken at different phases of a cyclic intrinsic movement of the body. In this case, an image database comprising a number of three-dimensional images of a periodically moving organ of the body, such as the heart for example, is generated prior to a catheter examination, with a movement signal (ECG, breathing signal) being recorded at the same time as the images. During the subsequent medical intervention, the spatial position of the instrument and also of a reference probe are determined by means of a locating device and the movement signal is recorded at the same time. By means of the movement signal, the relevant 3D image in terms of the movement phase of the organ of the body can then be selected from the image database and used for display purposes. One disadvantage of the known method is in particular the high outlay associated therewith.

WO 0016684 A discloses a system in accordance with the preamble of claims 1 and 8.

Against this background, it is an object of the present invention to provide means for the simpler navigation of an instrument in a vascular system, with compensation of cyclic intrinsic movements of the body.

This object is achieved by a navigation system having the features of claim 1 and by a navigation system having the features of claim 8. Advantageous refinements are contained in the dependent claims.

The navigation system according to the invention is used to navigate a catheter in a vascular system which is subject to (at least) one cyclic intrinsic movement. The intrinsic movement may in particular be caused by the heartbeat and/or the breathing. Furthermore, the term "catheter" is to be understood in a broad sense and hereinafter encompasses any instrument which is to be moved in a controlled manner through the vascular system of a patient. The navigation system comprises the following components:
a) A locating device which is designed to record a temporal sequence of position signals, with each of the position signals indicating the spatial position of the catheter at the associated measurement time. In this connection, the term "position of the catheter" is to be understood to mean the spatial position *r*, expressed for example by Cartesian coordinates, of at least one selected point of the catheter, with the term "position" possibly also encompassing the orientation of the catheter expressed for example by three angles.
   Furthermore, the term "temporal sequence" refers to the fact that for each position signal the associated measurement time *t* is known as a temporal coordinate. The sequence therefore typically consists of spatial-temporal coordinates *(r*, *t).*
b) A data processing device which is coupled to the locating device and is designed to calculate, by filtering the abovementioned sequence of position signals, a trajectory of the catheter which is compensated with respect to the cyclic intrinsic movement considered. The "compensated trajectory" thus reflects the positions which the catheter would assume without the intrinsic movement of the vascular system. A filtering in the sense of the present application is defined by the recalculation of a data record based on an original data record and a predefined algorithm. Examples of preferred filtering methods are discussed with reference to the dependent claims.

The navigation system described has the advantage that it allows the monitoring of the movement of a catheter in a vascular system solely by using a locating device, where disruptive intrinsic movements of the body are compensated by a filtering of the measurement data obtained. The compensated trajectory obtained in this way can then be displayed for example on a static road map. In order to record these road maps, the patient has to be exposed to X-ray radiation and contrast agent just once or in any case only a few times. Moreover, during the intervention neither the continually repeated taking of X-ray images nor the measurement of other physiological parameters such as an ECG for example are absolutely necessary. Furthermore, the possible use of a single road map has the advantage of providing the treating physician with a continuous background image which allows him to concentrate fully on the catheter movement that is to be observed.

In a first embodiment of the navigation system, the filtering of the position signal sequence comprises the following steps:
a) Calculation of the frequency spectrum of the temporal sequence of the position signals, where the position signals are recorded as a function of the time (at which they are recorded). The frequency analysis thus examines in particular the temporal course of a location vector *r(t)* which indicates the measured position of the catheter at the respectively associated point in time.
b) Calculation of a filtered frequency spectrum by reducing the amplitude of the abovementioned calculated frequency spectrum of the position signal sequence, where the amplitude is reduced in one or more frequency windows that are characteristic of the cyclic intrinsic movement considered. If the cyclic intrinsic movement that is to be compensated is the heartbeat, for example, the characteristic frequency windows are at the heartbeat frequency and multiples thereof. This is because movement parts of the continuously measured trajectory of the catheter which occur at these frequencies are very probably caused by the heartbeat. By reducing the amplitude of these parts, the effect of the heartbeat on the trajectory spectrum of the catheter can therefore be eliminated. The amplitude is preferably reduced to values that are comparable with the amplitude at the edges of the characteristic frequency window considered. Amplitude maxima caused by the cyclic intrinsic movement are thereby leveled out.
c) Finally, the synthesis (composition) of a compensated trajectory from the spectrum filtered according to step b) takes place. By suppressing the amplitudes in the characteristic frequency windows, the compensated trajectory no longer contains the deflections caused by the cyclic intrinsic movement of the vascular system.

In the above-described filtering method, the characteristic frequency windows mentioned therein are preferably obtained from the position of local maxima of the frequency spectrum calculated in step a). The local maxima are then eliminated or leveled out in step b). Deflections in the trajectory of a catheter that are caused by a cyclic intrinsic movement of the body such as the heartbeat for example usually lead to maxima in the frequency spectrum at the heart frequency and multiples thereof. This has the advantage that the disruptive intrinsic movement can be recognized in the shape of the frequency spectrum and accordingly compensated.

In an alternative embodiment not part of the invention, of the filtering of a position signal sequence, a sliding center of the position signals of the sequence is calculated over a time interval that is characteristic of the cyclic intrinsic movement of the vascular system considered. In particular, the time interval may be a period duration of the movement cycle, for example the duration of a heartbeat. All position signals from such a time interval are thus combined in the compensated trajectory to give a center. This type of filtering is based on the fact that any point of the vascular system during a cycle of the intrinsic movement considered is moved on an essentially closed curve if no other superposed movements take place or these are already compensated in some other way. The centre of such a curve therefore represents a characteristic position that has been cleaned of the cyclic intrinsic movement, and this position can be regarded for navigation purposes as the position of the catheter in the vascular system. Similar considerations apply with respect to the cyclically varying orientation of a catheter, so that in this case too the formation of a center supplies a useful mean value.

According to one development of the navigation system, the latter contains a measuring system for recording an electrocardiogram (ECG), said measuring system being coupled to the data processing device. As already mentioned, the heartbeat is a particularly important cyclic intrinsic movement of a vascular system that has to be compensated. Monitoring of the ECG therefore gives the possibility of increasing the precision of the heartbeat compensation and of additionally using other known methods for heartbeat compensation. In conjunction with the abovementioned spectral filtering of the position signal sequence, an ECG may also be used to calculate the characteristic frequency windows in which the amplitude of the spectrum is to be reduced. In particular, the current heart frequency can be determined or predicted from the ECG so that the frequency window can be placed accordingly. Furthermore, monitoring of the ECG can be used to recognize extra systoles which differ from the normal rhythm of the heart. Such irregular extra movements usually disrupt the described calculation of a compensated trajectory, since this is primarily based on periodicities of the respective intrinsic movement of the vascular system which remain constant or change only slowly. Position signals recorded during an extra systole are therefore preferably removed from the position signal sequence in order not to adversely affect the calculation of the compensated trajectory.

As mentioned above, the heartbeat and breathing are the most important cyclic intrinsic movements of the body which make navigation of a catheter more difficult. In one preferred refinement of the navigation system, the cyclic intrinsic movement considered and compensated in the abovementioned manner is caused by the heartbeat. The compensated trajectory thus still contains disruptions on account of breathing. The data processing device is therefore preferably also designed to correct the compensated trajectory with respect to an intrinsic movement of the vascular system caused by breathing.

In this respect, in the event of an optional type of breathing correction the data processing device is designed to carry out the following steps:
a) Calculation of a movement pattern caused by breathing from part-sections of the compensated trajectory in which there has been no movement (no advance or retreat) of the catheter relative to the vascular system. If required, the compensated trajectory is **characterized in that** the movement parts caused by the heartbeat are already eliminated. In one part-section - that is to say a group of temporally successive position signals - of the compensated trajectory in which it is known that there has been no relative movement of the catheter in the vascular system, any location change in the compensated trajectory must therefore be caused by the breathing movement. Such part-sections are therefore suitable for recognizing the movement pattern present at the associated location of the vascular system and caused by breathing. The movement pattern can in this case be described for example by periodically time-dependent difference vectors which on the basis of a spatially constant reference point allocate each point in time to a predefined point of the vascular system on the compensated trajectory. Part-sections of the compensated trajectory in which there is no relative movement of the catheter are preferably detected by an additional device in the navigation system, which additional device records for example an advance or a retreat of the catheter in the vascular system and provides this information in addition to the recorded sequence of the position signals.
b) Correction of the compensated trajectory by subtracting the movement pattern calculated in step a).

According to one variant for correcting the breathing movement, the data processing device is designed to correct the compensated trajectory by applying a spatial extrapolation filter (e.g. Kalman filter) on the basis of a previously determined movement pattern.

According to another development of the navigation system, the latter may comprise a breathing sensor that is coupled to the data processing device. A breathing sensor supplies a signal which represents a characteristic point in time of the breathing cycle and/or the phase profile of the breathing cycle. With the aid of this signal, the breathing correction of the trajectory can be compared and thus made even more precise, or alternative methods for correcting breathing movements may be used.

The description furthermore relates to a method of navigating a catheter in a vascular system which is subject to at least one cyclic intrinsic movement (in particular the heartbeat or breathing). The method comprises the following steps:
a) Recording of a temporal sequence of position signals which indicate the respective spatial position (position and possibly orientation) of the catheter.
b) Calculation, by filtering the sequence of position signals, of a trajectory of the catheter that has been compensated with respect to the cyclic intrinsic movement.

The method described thus comprises in a general manner the steps that can be carried out by the navigation system described above. For details regarding the refinement, advantages and developments of the method, reference should therefore be made to the above explanations.

The invention will be further described with reference to examples of embodiments shown in the drawings to which, however, the invention is not restricted.
Fig. 1 schematically shows the use of a navigation system according to the invention in a catheter examination of coronary vessels.
Fig. 2 shows a spatial representation of a measured catheter trajectory T₀ and of a trajectory T_{c} that has been compensated by spectral filtering with respect to the heartbeat.
Fig. 3 shows the frequency spectrum of the trajectories of Fig. 2.
Fig. 4 shows the trajectory T₀, formed by breathing movement and heartbeat, of a catheter that is stationary relative to a vessel section (left), the trajectory T₁ of the catheter that has been corrected with respect to the breathing movement (center), and the calculated center T₂ of the last-mentioned trajectory (right).
Fig. 5 shows a spatial representation of a measured catheter trajectory T₀ and of a trajectory T_{c} that has been compensated by center calculation with respect to the heartbeat.

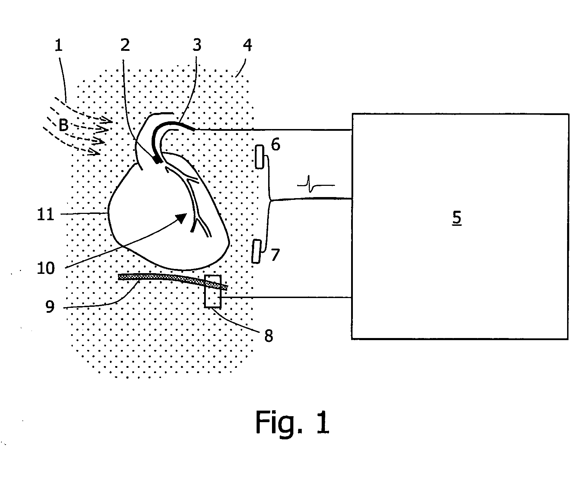

Fig. 1 schematically shows the components of a navigation system according to the invention, by means of which a catheter 3 can be guided in the vascular system 10 of a patient in order for example to examine the coronary vessels of the heart 11. At the tip of the catheter 3 there is a magnetic field probe 2 which can be used to measure the strength and direction of a magnetic field 1 impressed on the space by a field generator (not shown). The resulting measured signal is forwarded to a data processing device 5 (computer), where information about the current absolute spatial position of the probe 2 and hence of the catheter 3 can be obtained from the measured signals. The probe 2 is thus a locating device which supplies a temporal sequence of position signals relating to the current position r(t) = (x(t), y(t), z(t)) and orientation φ(t) = (α(t), β(t), γ(t)) (α = yaw angle, β = angle of inclination, γ = roll angle, t = time) of the catheter 3. Instead of the position determination with the aid of a magnetic field 1 which is shown by way of example, other methods could of course also be used to determine the current position and possibly orientation of the catheter 3.

The navigation system furthermore comprises electrodes 6, 7 for recording an electrocardiogram and also a breathing sensor 8 which monitors for example the movement of the diaphragm 9. The signals from these sensors are likewise passed to the data processing device 5.

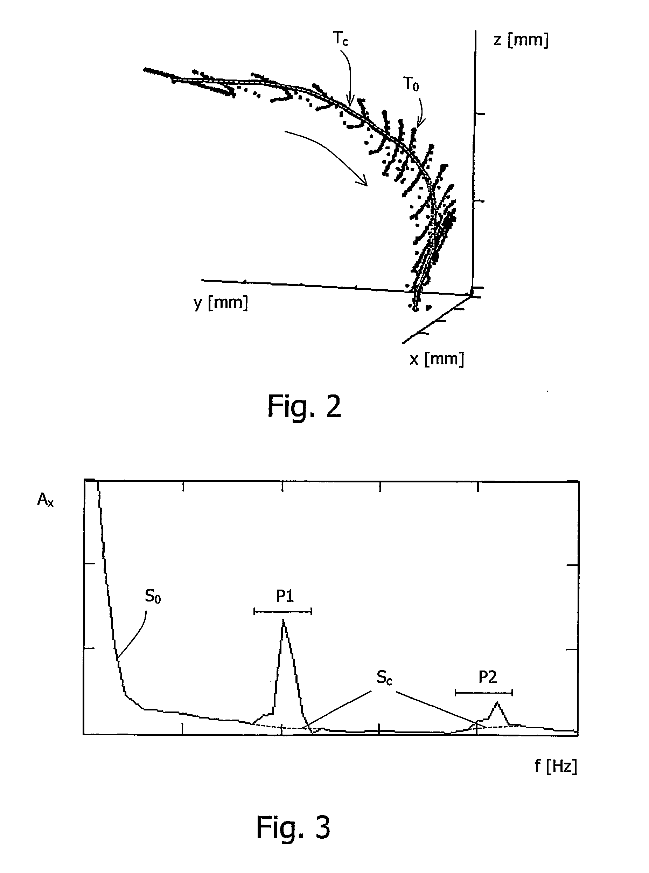

In order to minimize the exposure of the patient to X-ray radiation and contrast agent injections, it is endeavored that the movement of the catheter 3 be monitored on a few static X-ray images of the vascular system 10, known as "road maps". In this case, however, the intrinsic movement of the vascular system 10 caused by the heartbeat and by a cyclic movement of the thorax cavity 4 on account of the breathing must be taken into account and compensated. In this respect, Fig. 2 shows the profile of a trajectory T₀ in a spatial coordinate system with the axes *x, y* and *z,* said trajectory being formed from the sequence of position signals *r(t)* supplied by the magnetic sensor 2. The trajectory T₀ shows, in the center, a movement running in the direction of the arrow which corresponds to the advance of the catheter along the vessel, this advance being performed by the physician. However, deflections caused by the heartbeat are superposed on this movement (these being shown in the figure as transverse deflections relative to the vessel). Two methods of compensating these deflections are described below with reference to Figs. 3 to 5.

Fig. 3 shows, by way of example, the spectrum (amplitude Aₓ over frequency f) of the x coordinate of the trajectory T₀ of Fig. 2. This means that the x coordinate of the trajectory T₀ is plotted as a function of the time t, with a value x(t) being the measured (or interpolated) value of the x coordinate at the measuring time (or interpolation time) t. In the section shown, the frequency spectrum S₀ of this time function t → x(t) has two local maxima P1 and P2. These maxima lie in characteristic frequency windows which correspond to the heartbeat frequency or multiples thereof, and result from the movement of the catheter 3 caused by the heartbeat. In order to eliminate the effect of the heartbeat on the measured trajectory T₀, a compensated spectrum S_{c} is calculated which no longer contains said local maxima P1, P2. In the simplest case, the maxima may be eliminated by a linear interpolation of the amplitude values which are adjacent to the maximum to the right and left. This gives the profile of the corrected spectrum S_{c} which is shown in dashed line.

Following determination of the corrected spectrum S_{c} in the frequency range, this spectrum can be reverse-transformed into the position space, resulting in the compensated trajectory T_{c} shown in dashed line in Fig. 2.

The abovementioned frequency filtering gives the best results for compensation of the heartbeat. Since the frequencies of the breathing movement are in the same order of magnitude as the advance frequency of the catheter, frequency filtering for compensating breathing movements is usually less successful. In order also to remove the breathing movements from the trajectory T_{c} compensated with respect to the heartbeat, therefore, use is preferably made of other methods. For example, the intrinsic movement of the vascular system may be recorded locally by a marker that is stationary in the vascular system or by an electromagnetically located sensor (e.g. similar to the sensor 2 of Fig. 1 used to locate the catheter). Following removal of the heart movement from this intrinsic movement (e.g. by the same frequency filtering), the movement pattern of solely the breathing movement remains, which can then in turn be subtracted from the compensated trajectory of the catheter position. A marker or sensor could also be arranged at a position of the heart, for example the ostium of the left coronary artery, where it is essentially moved only by breathing movements and not by the heartbeat. Furthermore, it is possible to use, from the compensated trajectory T_{c}, in order to determine the movement pattern of the breathing, those sections during which it is known that the catheter 3 has not been pushed forward or back in the vascular system. Such a section of the trajectory T_{c} which has been compensated with respect to the heartbeat then describes the effect of the breathing movement alone.

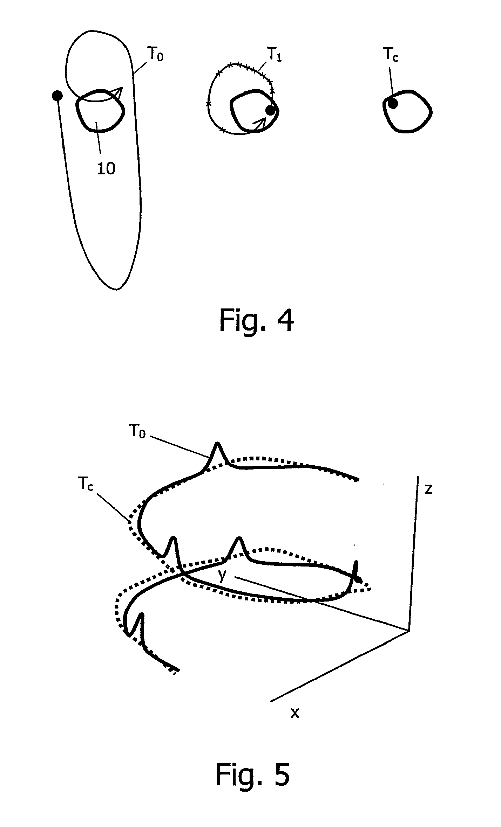

Fig. 4 shows an alternative method of compensating the heartbeat. The figure shows a cross section through a vessel 10 in the rest position thereof. Furthermore, a section of the trajectory T₀ on which a catheter (not shown) resting in the vessel section moves on account of the vessel movement caused by breathing and heartbeat is shown in the left-hand part of the figure. Since breathing and heartbeat are not synchronous, this section of the trajectory is not closed. The length of the trajectory should correspond approximately to the duration of a heartbeat.

In the central part of Fig. 4 there is shown a corrected trajectory T₁ which results from the measured trajectory T₀ shown on the left as a result of correction with respect to the breathing movement. Details regarding possible methods for breathing compensation have already been discussed above with reference to Fig. 3. The trajectory T₁ which can then only be attributed to the heartbeat has a significantly smaller amplitude and is more or less closed. The (geometric) center of this trajectory T₁ can then be calculated and defined as the current catheter position that has been cleaned of the heart movement. The calculated center is shown as part of the resulting compensated trajectory T_{c} in the right-hand part of Fig. 4. A similar center calculation is in principle also possible in respect of an (abstract) trajectory of catheter orientations in a vector space of orientations φ.

Usually, the trajectories T₀ and T₁ will firstly consist of individually measured position signals which are shown by crosses in the central part of Fig. 4. In order to calculate the center, these discrete points should be supplemented (i.e. interpolated) to form a continuous part of a curve, so that the real "geometric" or, if the part of a curve is considered as having mass, "physical" center can be determined by integration along the part of the curve. The "mass" of a measured value can in this case be defined by the measurement certainty, so that "good" measured values are used to a greater extent to form the center of the trajectory than those whose measured value is less reliable.

Filtering by center calculation may in principle also be used to compensate the breathing movement. However, since a breathing cycle lasts considerably longer than a heart cycle, the basic time window for data averaging is accordingly greater, which in turn leads to interference with the voluntary catheter movement by the examining physician.

Fig. 5 shows, in a representation corresponding to Fig. 2, an exemplary original trajectory T₀ of position signals and also the compensated trajectory T_{c} determined therefrom by means of the center method.

The sensors 6, 7 for an ECG and 8 for breathing that are shown in Fig. 1 may be used to further improve the above-described compensation method. In particular, changes in the heartbeat and/or breathing frequency can be determined by the sensors. If, for example, an extra systole that deviates from the basic heart frequency occurs, then the associated position data of the catheter can be excluded from the processing. Alternatively, the catheter position in this time window may also be determined using a model-based filter, for example a Kalman filter (cf. R.E. Kalman, "A New Approach to Linear Filtering and Prediction Problems", Transactions of the ASME - Journal of Basic Engineering, 82 (Series D), 35-45, 1960; P.S. Maybeck: "Stochastic models, estimation, and control, Vol. I", Academic Press, 1979). A Kalman filter may also be used to predict the movement caused by breathing. In this case, use is only made of measurements of the catheter position which all belong to a determined defined position in the breathing cycle. Such positions may for example by detected by an additional breathing sensor or the local movement pattern.

## Claims

1. A navigation system for navigating a catheter (3) in a vascular system (10) which is subject to a cyclic intrinsic movement, comprising
a) a locating device (2) for recording a temporal sequence (To) of position signals which indicate the respective spatial position of the catheter (3);
b) a data processing device (5) which is coupled to the locating device (2) and is designed to calculate, by filtering the sequence (To) of position signals, a trajectory (Tc) of the catheter (3) that is compensated with respect to the cyclic intrinsic movement,
**characterized in that** the filtering comprises the following steps:
a) calculation of the frequency spectrum (So) of the position signals of the sequence (To) that have been recorded as a function of time;
b) calculation of a filtered spectrum (Sc) by reducing the amplitude (A) of said frequency spectrum (So) in at least one frequency window that is characteristic of the cyclic intrinsic movement;
c) synthesis of the compensated trajectory (Tc) from the filtered spectrum (Sc).

2. A navigation system as claimed in claim 1, **characterized in that** the characteristic frequency windows are obtained from the position of local maxima (P1, P2) of the frequency spectrum (So) calculated in step a).

3. A navigation system as claimed in claim 1, **characterized in that** it has a measuring system (6,7) for recording an electrocardiogram, said measuring system being coupled to the data processing device (5).

4. A navigation system as claimed in claim 1, **characterized in that** the cyclic intrinsic movement is caused by the heartbeat, and **in that** the data processing device (5) is furthermore designed to correct the compensated trajectory (Tc) with respect to an intrinsic movement caused by breathing.

5. A navigation system as claimed in claim 4, **characterized in that** the data processing device (5) is designed to carry out the following steps: a) calculation of a movement pattern caused by breathing from part-sections of the compensated trajectory (Tc) in which there has been no movement of the catheter (3) relative to the vascular system (10); b) correction of the compensated trajectory (Te) by subtracting the calculated movement pattern.

6. A navigation system as claimed in claim 4, **characterized in that** the data processing device (5) is designed to correct the compensated trajectory (Tc) by applying an extrapolation filter on the basis of a previously determined movement pattern.

7. A navigation system as claimed in claim 1, **characterized in that** it has a breathing sensor (8) that is coupled to the data processing device (5).

8. A navigation system for navigating a catheter (3) in a vascular system (10) which is subject to a cyclic intrinsic movement, comprising
a) a locating device (2) for recording a temporal sequence (To) of position signals which indicate the respective spatial position of the catheter (3);
b) a data processing device (5) which is coupled to the locating device (2) and is designed to calculate, by filtering the sequence (To) of position signals, a trajectory (Tc) of the catheter (3) that is compensated with respect to the cyclic intrinsic movement,
wherein the cyclic intrinsic movement is caused by the heartbeat, and in that the data processing device (5) is furthermore designed to correct the compensated trajectory (Tc) with respect to an intrinsic movement caused by breathing,
**characterized in that** the data processing device (5) is designed to correct the compensated trajectory (Tc) by applying an extrapolation filter on the basis of a previously determined movement pattern.

## Patentansprüche

1. Navigationssystem zum Navigieren eines Katheters (3) in einem Gefäßsystem (10), das einer zyklischen Eigenbewegung unterliegt, wobei es Folgendes umfasst:
a) eine Ortungseinrichtung (2) zum Aufzeichnen einer zeitlichen Sequenz (To) von Positionssignalen, die die entsprechende räumliche Position des Katheters (3) angeben,
b) eine Datenverarbeitungseinrichtung (5), die mit der Ortungseinrichtung (2) gekoppelt und derart ausgelegt ist, dass sie durch Filtern der Sequenz (To) mit Positionssignalen eine Trajektorie (Tc) des Katheters (3) berechnet, die mit Bezug auf die zyklische Eigenbewegung kompensiert wird,
**dadurch gekennzeichnet, dass** das Filtern die folgenden Schritte umfasst:
a) Berechnen des Frequenzspektrums (So) der Positionssignale der Sequenz (To), die als Funktion der Zeit aufgezeichnet wurden,
b) Berechnen eines gefilterten Spektrums (Sc) durch Reduzieren der Amplitude (A) des genannten Frequenzspektrums (So) in mindestens einem Frequenzfenster, das für die zyklische Eigenbewegung charakteristisch ist,
c) Synthese der kompensierten Trajektorie (Tc) aus dem gefilterten Spektrum (Sc).

2. Navigationssystem nach Anspruch 1, **dadurch gekennzeichnet, dass** die charakteristischen Frequenzfenster aus der Position von lokalen Maximalwerten (P1, P2) des in Schritt a) berechneten Frequenzspektrums (So) erzielt werden.

3. Navigationssystem nach Anspruch 1, **dadurch gekennzeichnet, dass** es ein Messsystem (6, 7) zum Aufzeichnen eines Elektrokardiogramms aufweist, wobei das genannte Messsystem mit der Datenverarbeitungseinrichtung (5) gekoppelt ist.

4. Navigationssystem nach Anspruch 1, **dadurch gekennzeichnet, dass** die zyklische Eigenbewegung durch den Herzschlag verursacht wird, und dass die Datenverarbeitungseinrichtung (5) ferner derart ausgelegt ist, dass sie die kompensierte Trajektorie (Tc) in Bezug auf eine durch die Atmung verursachte Eigenbewegung korrigiert.

5. Navigationssystem nach Anspruch 4, **dadurch gekennzeichnet, dass** die Datenverarbeitungseinrichtung (5) derart ausgelegt ist, dass sie die folgenden Schritte durchführt:
a) Berechnen eines durch Atmung verursachten Bewegungsmusters aus Teilabschnitten der kompensierten Trajektorie (Tc), in der keine Bewegung des Katheters (3) in Bezug auf das Gefäßsystem (10) stattgefunden hat,
b) Korrigieren der kompensierten Trajektorie (Tc) durch Subtrahieren des berechneten Bewegungsmusters.

6. Navigationssystem nach Anspruch 4, **dadurch gekennzeichnet, dass** die Datenverarbeitungseinrichtung (5) derart ausgelegt ist, dass sie die kompensierte Trajektorie (Tc) korrigiert, indem sie auf der Grundlage eines vorher ermittelten Bewegungsmusters ein Extrapolationsfilter anwendet.

7. Navigationssystem nach Anspruch 1, **dadurch gekennzeichnet, dass** es über einen Atmungssensor (8) verfügt, der mit der Datenverarbeitungseinrichtung (5) gekoppelt ist.

8. Navigationssystem zum Navigieren eines Katheters (3) in einem Gefäßsystem (10), das einer zyklischen Eigenbewegung unterliegt, wobei es Folgendes umfasst:
a) eine Ortungseinrichtung (2) zum Aufzeichnen einer zeitlichen Sequenz (To) mit Positionssignalen, die die entsprechende räumliche Position des Katheters (3) angeben,
b) eine Datenverarbeitungseinrichtung (5), die mit der Ortungseinrichtung (2) gekoppelt und derart ausgelegt ist, dass sie durch Filtern der Sequenz (To) mit Positionssignalen eine Trajektorie (Tc) des Katheters (3) berechnet, die mit Bezug auf die zyklische Eigenbewegung kompensiert wird,
wobei die zyklische Eigenbewegung durch den Herzschlag verursacht wird, und die Datenverarbeitungseinrichtung (5) ferner derart ausgelegt ist, dass sie die kompensierte Trajektorie (Tc) in Bezug auf eine durch die Atmung verursachte Eigenbewegung korrigiert,
**dadurch gekennzeichnet, dass** die Datenverarbeitungseinrichtung (5) derart ausgelegt ist, dass sie die kompensierte Trajektorie (Tc) korrigiert, indem sie auf der Grundlage eines vorher ermittelten Bewegungsmusters ein Extrapolationsfilter anwendet.

## Revendications

1. Système de navigation pour faire naviguer un cathéter (3) dans un système vasculaire (10) qui est sujet à un mouvement intrinsèque cyclique, comprenant :
a) un dispositif de localisation (2) pour enregistrer une séquence temporelle (To) de signaux de position qui indiquent la position spatiale respective du cathéter (3) ;
b) un dispositif de traitement de données (5) qui est couplé au dispositif de localisation (2) et est conçu pour calculer, par filtrage de la séquence (To) de signaux de position, une trajectoire (Tc) du cathéter (3) qui est compensée par rapport au mouvement intrinsèque cyclique,
**caractérisé en ce que** le filtrage comprend les étapes suivantes
a) calcul du spectre de fréquence (So) des signaux de position de la séquence (To) qui ont été enregistrés en fonction du temps ;
b) calcul d'un spectre filtré (Sc) par réduction de l'amplitude (A) dudit spectre de fréquence (So) dans au moins une fenêtre de fréquence qui est caractéristique du mouvement intrinsèque cyclique ;
c) synthèse de la trajectoire compensée (Tc) à partir du spectre filtré (Sc).

2. Système de navigation selon la revendication 1, **caractérisé en ce que** les fenêtres de fréquence caractéristiques sont obtenues à partir de la position de maxima locaux (P1, P2) du spectre de fréquence (So) calculée à l'étape a).

3. Système de navigation selon la revendication 1, **caractérisé en ce qu'**il comporte un système de mesure (6, 7) pour enregistrer un électrocardiogramme, ledit système de mesure étant couplé au dispositif de traitement de données (5).

4. Système de navigation selon la revendication 1, **caractérisé en ce que** le mouvement intrinsèque cyclique est provoqué par le battement cardiaque, et **en ce que** le dispositif de traitement de données (5) est de plus conçu pour corriger la trajectoire compensée (Tc) par rapport à un mouvement intrinsèque provoqué par la respiration.

5. Système de navigation selon la revendication 4, **caractérisé en ce que** le dispositif de traitement de données (5) est conçu pour réaliser les étapes suivantes :
a) calcul du motif de mouvement provoqué par la respiration provenant de sections partielles de la trajectoire compensée (Tc) dans lesquelles il n'y a pas de mouvement du cathéter (3) par rapport au système vasculaire (10) ;
b) correction de la trajectoire compensée (Te) par soustraction du motif de mouvement calculé.

6. Système de navigation selon la revendication 4, **caractérisé en ce que** le dispositif de traitement de données (5) est conçu pour corriger la trajectoire compensée (Tc) en appliquant un filtre d'extrapolation en se basant sur un motif de mouvement antérieurement déterminé.

7. Système de navigation selon la revendication 1, **caractérisé en ce qu'**il comporte un capteur de respiration (8) qui est couplé au dispositif de traitement de données (5).

8. Système de navigation pour faire naviguer un cathéter (3) dans un système vasculaire (10) qui est sujet à un mouvement intrinsèque cyclique, comprenant
a) un dispositif de localisation (2) pour enregistrer une séquence temporelle (To) de signaux de position qui indiquent la position spatiale respective du cathéter (3) ;
b) un dispositif de traitement de données (5) qui est couplé au dispositif de localisation (2) et est conçu pour calculer, par filtrage de la séquence (To) de signaux de position, une trajectoire (Tc) du cathéter (3) qui est compensée par rapport au mouvement intrinsèque cyclique,
dans lequel le mouvement intrinsèque cyclique est provoqué par le battement cardiaque, et en ce que le dispositif de traitement de données (5) est de plus conçu pour corriger la trajectoire compensée (Tc) par rapport à un mouvement intrinsèque provoqué par la respiration,
**caractérisé en ce que** le dispositif de traitement de données (5) est conçu pour corriger la trajectoire compensée (Tc) en appliquant un filtre d'extrapolation en se basant sur un motif de mouvement antérieurement déterminé.
